# EUROPÄISCHE PATENTSCHRIFT

(11) **EP 0 732 339 B1**
(45) Veröffentlichungstag und Bekanntmachung des Hinweises auf die Patenterteilung: **24.04.2002**
(21) Anmeldenummer: 96100385.2
(22) Anmeldetag: 12.12.1991
(51) Int. Cl.: C07K 14/16, A61K 38/55

(54) **Peptide des HIV-gag Proteins, ihre Herstellung und Verwendung**
Peptides of the HIV-gag protein, their preparation and use
Peptides de la protéine gag d'HIV, leur procédé de préparation et leur utilisation

(30) Priorität: 14.12.1990 DE 4039925
(43) Veröffentlichungstag der Anmeldung: 18.09.1996
(62) Teilanmeldung aus: 91121362.7
(73) Patentinhaber: Chiron Behring Gmbh & Co., 35006 Marburg (DE)
(72) Erfinder: Niedrig, Matthias, Dr., 12203 Berlin (DE); Wolf, Hans, Prof., 82319 Starnberg (DE); Modrow, Susanne, Dr., 93177 Altenthann (DE)
(74) Vertreter: Hallybone, Huw George

(56) Entgegenhaltungen:
- EP-A- 0 284 587
- EP-A- 0 290 893
- EP-A- 0 373 070
- WO-A-86/06414
- WO-A-90/13564
- WO-A-91/09869
- WO-A-91/13360

## Beschreibung

Die Erfindung betrifft ausgewählte Peptide der HIV-gag-Sequenz, die eine Inhibition der Virussynthese bewirken. Bei Testung von 41 sequenziellen Peptiden auf Hemmung der HIV-Vermehrung wurde gefunden, daß die überlappenden Peptide 4 und 5 mit der Aminosäuresequenz WASRELERFAVNPGLLETSEGCRQ und NPGLLETSEGCRQILGQLQPSLQT aus dem p17 Submembranprotein und die beiden Peptide 28 und 29 mit den Aminosäuresequenzen ANPDCKTILKALGPAATLEEMMTA und AATLEEMMTACQGVGGPGHKA aus dem p24 Coreprotein die Vermehrung von HIV hemmen (überlappende Bereiche sind jeweils unterstrichen). Solche oder ähnliche Peptide, die mindestens die überlappenden Bereiche enthalten, sind als Arzneimittel zur Bekämpfung von HIV-Infektionen geeignet.

Die durch das HIV verursachte Erkrankung AIDS stellt eine große Anforderung an die wissenschaftliche Forschung bei der Entwicklung therapeutisch wirksamer Substanzen und neuer Vakzinen dar. Auch wenn sich die Forschung über das gesamte Spektrum möglicher Therapieansätze erstreckt, versprechen doch nur sehr wenige Substanzen Aussicht auf eine neue erfolgreiche Therapie. Bisher ist als einzige zugelassene anti-HIV wirksame Substanz^{R} Retrovir mit dem Wirkstoff Zidovudin der Firma Wellcome auf dem Markt. Dieses Nukleotidanalog, Azidothymidine (AZT) hemmt sehr wirkungsvoll in vitro und in vivo die HIV-spezifische Reverse Transkriptase, ist jedoch nicht frei von nachteiligen Eigenschaften. Bisher bestehen keine Alternativen zu der AZT-Therapie.

Untersuchungen über die HIV-Virusstruktur und die Rolle der verschiedenen Virusstruckturproteine bei der Virusreifung führten zu der Erkenntnis, daß sich hier mehrere Ansätze für eine wirkungsvolle Inhibition der Virussynthese bieten, wobei die Inhibition der viralen Protease durch Chemotherapeutika nur ein Beispiel darstellt, das zur Zeit von verschiedenen Arbeitsgruppen bearbeitet wird. Die Tatsache, daß die gag-Sequenz zu den hochlonservierten Regionen im HIV-Genom zählt, läßt vermuten, daß es sich hierbei um ein für die Virusvermehrung sehr wichtiges Protein handelt; die geringen Sequenzunterschiede bei verschiedenen HIV-Isolaten unterstützen diese Vermutung (siehe Tab. 1). Der Vorgang der Virussynthese wird eingeleitet durch die gag-Proteinsynthese und die notwendige Myristinylierung des gag-Proteins. Der weitere Zusammenbau findet an der Lipidmembran der infizierten Zelle statt. Durch aneinanderlagern von gag-Proteinen kommt es zur Ansstütpung der Zeilmembran und zur Abschnürung ("Budding") von "Teilchen" bzw. unreifen Viren. Daß dieser Vorgang auch abläuft, wenn nur das gag-Gen in die Zeite eingeschleust wird, konnte durch Untersuchungen mit rekombinanten gag-Sequenzen in Vaccinia-bzw. Baculovectoren gezeigt werden (Karacostas et al. (1989) Human immunodeficiency virus-like particles produced by vaccinia viris expression vector. Proc. Natl. Acad. Sci. USA, Vol. 86, 8965-8967; Gheysen et al. (1989) Assembly and release of HIV-1 precursor prgag55 virus-like particles from recombinant baculovirus-infected insect cells. Cell 59, 103-112). Andere Publikationen belegen, daß es innerhalb der gag-Sequenz für das "Processing" wichtige Bereiche gibt. Durch Einführung gezielter Mutationen innerhalb der gag-Sequenz, wurden mehrere für das "Processing" relevante Bereiche identifiziert (Göttlinger et al. (1990) Role of Capsid precursor processing and myristoylation in morphogenesis and infectivity of human immunodeficiency virus type 1. Proc. Natl. Acad. Sci. USA, Vol. 86, 5781-5785).

Die internationale Patentanmeldung WO 86/06414 beschreibt Peptide mit einer Länge von mindestens 5 Aminosäuren , die in spezifischen Bindungstests für die Messungen von Antikörper gegen das LAV/HTLV-III Virus und für die Messung von LAV/HTLV-III Antigen oder als Immunogen Verwendung können.

Allerdings gab es keinerlei Hinweise darauf, daß bestimmte Bereiche der gag-Sequenz für Peptide kodieren, die die Virussynthese von HIV hemmen.

Aus der internationalen Patentanmeldung WO91/13360, die Stand der Technik gemäß Artikel 54(3) EPÜ darstellt, ist ein Peptid mit der Sequenz KALGPAATLEEMMTACQ bekannt, das mit Antikörpern reagieren kann, die gegen eine einem Epitop bzw. Epitopenclustern des Proteins p24 in HIV-1 entsprechenden oder damit eng verwandten Aminosäuresequenz erzeugt wurden.

In den der Erfindung zugrunde liegenden Versuchen wurden 41 einander überlappende synthetische Peptide der HIV-1 gag Sequenz auf ihre die Virussynthese hemmende Funktion in einem in vitro Test untersucht. Die 24 Aminosäuren langen Peptide wurden analog der von Ratner et al. (Complete nucleotide sequence of the AIDS virus, HTLV-III, Nature, 313, 277-284 (1985)) publizierten Sequenz synthetisiert.

Diese Peptide wurden in unterschiedlichen Konzentrationen (200-40 µg/ml) auf frisch infizierte Jurkat Zellen gegeben. Die Infektion erfolgte mit 100-1000 TCID₅₀.

Die Analyse der Infektion erfolgte durch mikroskopische Beurteilung und Untersuchung des Überstandes auf Gehalt an infektiösem HIV. Hierfür wurde Zellüberstand auf nicht infizierte Jurkatzellen gegeben. Nach zwei Wochen Inkubation wurde diese Nachweiszellkultur mikroskopisch und durch Reverse Transkriptase Assay auf HIV untersucht. Hierbei wurde gefunden, daß es zwei gag-Bereiche gibt, die einen hemmenden Effekt auf die Virussynthese ausüben. Diese beiden Bereiche sind jeweils durch zwei Peptide repräsentiert, der erste (Peptid 4+5) liegt innerhalb des p17, der zweite (Peptid 28+29) innerhalb des p24.

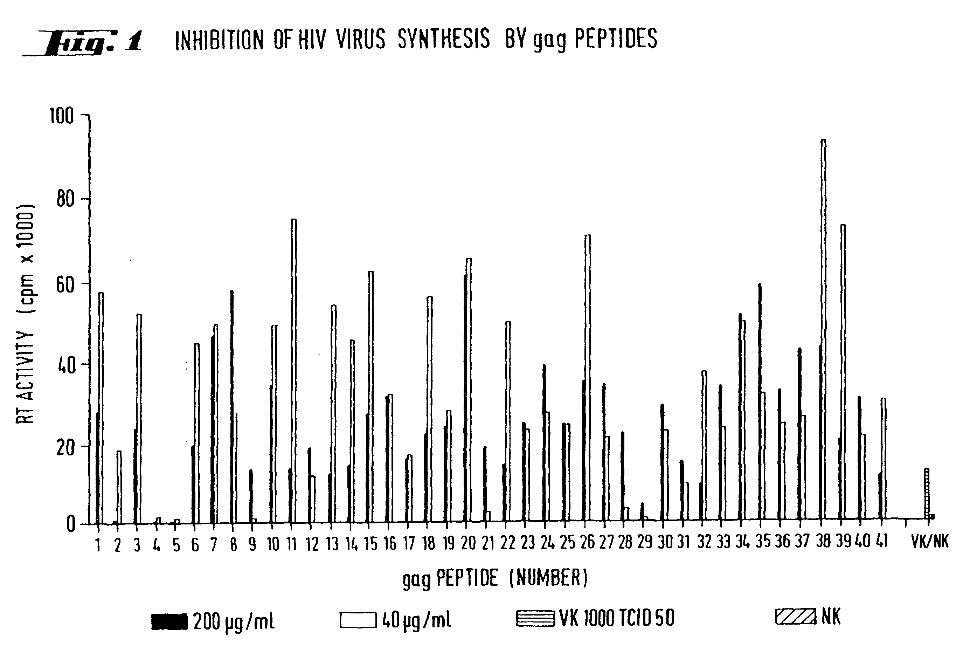

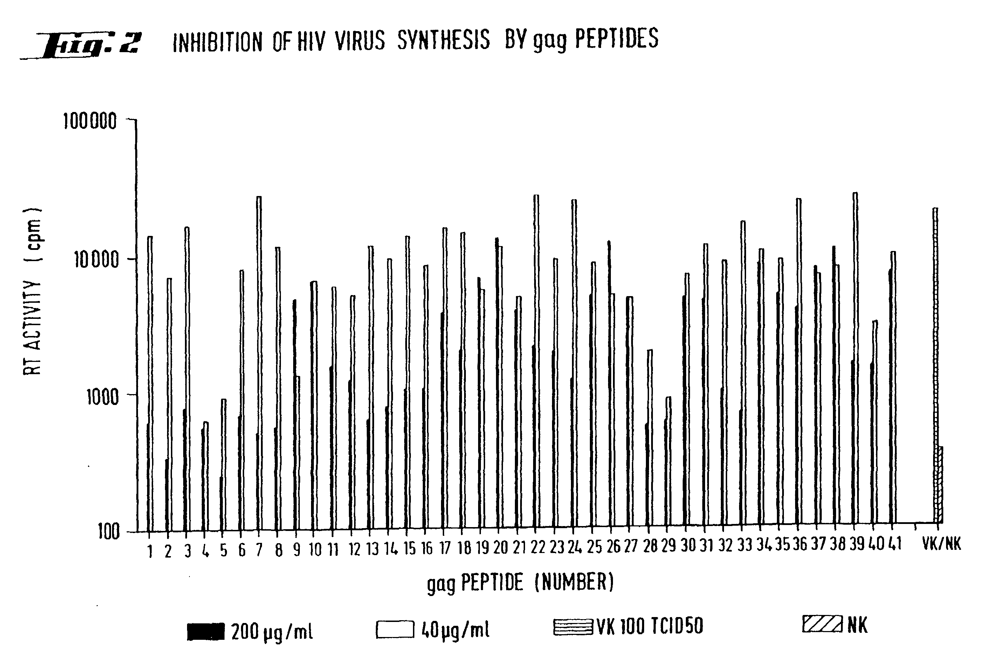

Wie aus Tab. 4 ersichtlich, läßt sich in HIV-infizierten Zellen nach Behandlung mit diesen Peptiden kein p24 nachweisen. Der Überstand dieser Zellkulturen enthält kein infektiöses HIV, daß zur Infektion einer Kontrollkultur in der Lage wäre. Die Figuren 1 und 2 geben die Reverse Transkriptase (RT) Aktivität der beiden Nachweiszellkulturen wieder. Die vier ausgewählten Peptide vermögen die HIV-Synthese in Konzentrationen von 200-40 µg/ml sehr wirkungsvoll zu inhibieren. (Einzelheiten zum Nachweis der HIV-Inhibition siehe Beispiele).

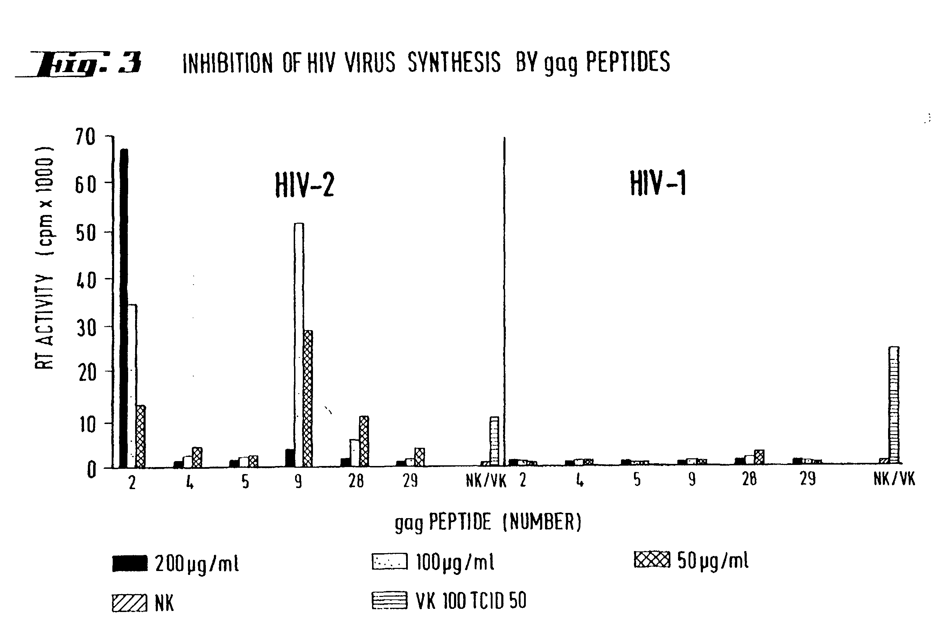

Der Einfluß von unterschiedlichen Konzentrationen an infektiösem Virus auf den Versuchsablauf ist relativ gering und spiegelt sich hauptsächlich in der Höhe der gemessenen RT-Aktivität wieder (vgl. Versuch 1, Fig. 1; Versuch 2, Fig. 2). Auch die unterschiedlichen Konzentrationen an zugesetzten Peptiden haben keinen signifikanten Einfluß auf die Inhibition, was vermuten läßt, daß die Konzentration der zugegebenen Peptide noch weiter abgesenkt werden kann, ohne die Wirkung zu beeinflussen. Das Ergebnis des dritten Versuches ist in Fig. 3 wiedergegeben. Die hier dargestellten Reverse Transkriptase Aktivitäten sind nach zwei Wochen Inkubation der Nachweiszellkulturen aus dem Zellkulturüberstand nach fünffacher Konzentration gemessen. Auch hier zeigt sich der starke inhibierende Einfluß der Peptide 2, 4, 5, 9, 28, 29 auf die HIV-1 Synthese. Die Inhibition der HIV-2 Synthese durch die Peptide 4, 5, 28, 29 ist auch sehr deutlich zu erkennen. Dennoch scheinen hier die zugegebenen Peptide schwächer in ihrer inhibierenden Wirkung zu sein. So kommt es bei den Peptiden 2 und 9 zu keiner Inhibition, bei 28 ist nur die 200 µg/ml Dosis zu einer vollständigen Hemmung der Virussynthese in der Lage. Bei allen 41 untersuchten Petiden konnte nur für das Peptid Nr. eine Zelltoxizität festgestellt werden.

Die Erfindung betrifft folglich Peptide, die die Peptidsequenz AATLEEMMTA enthalten und nicht größer als 50 Aminosäuren, insbesondere nicht größer als 40 Aminosäuren, vor allem nicht größer als 30 Aminosäuren, vorzugsweise nicht größer als 20 Aminosäuren sind. Insbesondere bevorzugt sind Peptide, die die Peptide 4, 5, 28 oder 29, enthalten.

Eine weitere Ausführungsform der Erfindung sind Peptide, die die Peptidsequenz AATLEEMMTA enthalten, wobei die Sequenzen am N- und/oder am C-Terminus um bis zu 7 Aminosäuren, vorzugsweise um bis zu 4 Aminosäuren verkürzt werden können, ohne jedoch eine Länge von 6 Aminosäuren zu unterschreiten. Häufig ist es auch vorteilhaft, das erfindungsgemäße Peptid um eine oder mehrere Aminosäuren, beispielsweise Cystein, zu verlängern, um das Verknüpfen der Peptide untereinander oder an einen Träger zu erreichen.

Desweiteren umfaßt die Erfindung Derivate der Peptidsequenz AATLEEMMTA, bei denen eine oder mehrere der folgenden Substitutionen nach dem Fachmann bekannten Methoden durchgeführt werden können:
Asparagin gegen Glutamin, bzw. Glutamin gegen Asparagin, Prolin gegen Hydroxyprolin, Leucin gegen Isoleucin oder Norleucin, Glutaminsäure gegen Asparaginsäure, Threonin gegen Serin bzw. Serin gegen Threonin, Cystein gegen Serin, Arginin gegen Lysin, Alanin gegen Glycin und/oder Methionin gegen Norleucin. Bevorzugt ist der Ersatz einer natürlichen Aminosäure durch eine nicht natürliche Amino- säure, wie beispielsweise Hydroxyprolin oder Norleucin. Dadurch kann verhindert werden, daß körpereigene Proteasen das Derivat spalten, was im allgemeinen zu einer Verlängerung der Halbwertszeit führt. Auch können die Derivate eine verbesserte Löslichkeit und/oder eine bessere Resorption besitzen im Vergleich zu der erfindungsgemäßen Peptidsequenz.

Weitere Gegenstände der Erfindung sind die proteinchemische oder gentechnische Herstellung sowie die Verwendung des erfindungsgemäßen Peptids als Arzneimittel.

Vorzugsweise wird das erfindungsgemäße Peptid proteinchemisch hergestellt, beispielsweise nach BARANI, G. und MERRIFIELD, R.B. in "The Petides, Analysis, Synthesis and Biology", Vol. 2, Academic Press 1980, Ed. Erhard Gross, Johannes Meienhofer.

Die Erfindung ist in den Beispielen näher erläutert und in den Patentansprüchen enthalten.

### Beispiel 1: Allgemeine Grundlagen

**Zellen**. Es wurden permanent wachsende T-Lymphozyten (Jurkat- oder H9-Zellen) für die in vitro-Experimente verwendet. Als Medium wurde konventionelles RPMI 1640 mit 10% FKS, 2% NaHCO₃ (5%ig), 1% Penicillin/Streptavidinlösung und 2 mg/l Polybrene verwendet. Die Experimente wurden wahlweise in 96 Loch-Mikrotiterplatten (Nunc) oder 24 Loch-Platten (Nunc) durchgeführt.

**Viren**. Es wurde HIV-1 virus Stamm HTLV-IIIB und HIV-2 Stamm ROD verwendet.

**Peptidsynthese und Peptidreinigung**. Die Peptide wurden nach der von Ratner et al. (1985) publizierten HIV-1 Sequenz auf einem Syntheseautomaten (Milligen 9050, Milligen GmbH, Eschborn, FRG) unter Einsatz von Fmoc-geschützten Aminosäuren (Bachem AG, Heidelberg, FRG) hergestellt (Atherton et al. (1978): A mild procedure for solid phase peptide synthesis; Use of Fluorenylmethyloxycarbonyl amino acids: J_{R} Chem. Soc. Chem. Commun. 13, 539-540). Als Trägermaterial wurde jeweils Tentagelharz mit säurestabilem AM-Linker verwendet (Rapp-Polymere, Tübingen, FRG). Die Aminosäuren wurden jeweils vor der Kopplung in DMF gelöst und in Hydroxybenzotriazol-aktivierte Ester überführt. Zur Kopplung wurden Schnellsynthesezyklen mit 10-minütiger Raktionszeit eingesetzt. Die Fmoc-Gruppe wurde in Anschluß mit 20% Piperidin abgespalten. Die Vollständigkeit dieser Reaktion wurde fluorometrisch überprüft.

Nach vollendeter Synthese wurde das Harz mit dem geschützten Peptid in 50% TFA/DCM suspendiert. Als Scavenger wurden 1% Anisol, 1% m-Kresol, 1% Phenol und, falls Trp in der jeweiligen Sequenz enthalten war, 5% Mercaptoethanol zugesetzt. Die Bindung zum Harz und die Trt- und tBoc-Schutzgruppen wurden in 4-stündiger Inkubationszeit bei Raumtemperatur unter Argon-Schutzgas abgespalten. Zur Entfernung der Mtr-Schutzgruppe des Arg wurde das abgespaltene Peptid vom Harz abgetrennt, das Lösungsmittel im Rotationsverdampfer abgezogen, die verbleibende Festsubstanz in 100% incl. den übliche Scavenger (siehe oben) über Nacht inkubiert. Die entschützten Peptide wurden nach Abzug des Lösungsmittel in 50% Essigsäure gelöst, in einem großen Volumen eiskalter t-Butylethylether gefällt, mehrmals gewaschen und lyophilisiert. Die getrocknete Rohsubstanz wurde in 1,5% Aminobicarbonat aufgenommen. Unlösliche Bestandteile wurden abfiltriert. Das Filtrat wurde erneut getrocknet. Die Reinigung der Peptide erfolgte auf einer halbpräperativen Propep Reversed Phase HPLC-Säule (C₂/C₁₈ Copolymer,

Pharmacia/LKB, Freiburg, FRG), wobei zur Elution üblicherweise Gradienten von 0-70% Acetonitril in 0,1% TFA unter Heliumbegasung eingesetzt wurden. Die Sequenzen der gereinigten Peptide wurden auf einem Gasphasensequenzer (Applied Biosystems, Westerstadt, FRG) überprüft. Die eingesetzten Peptide entsprechen den in Tab. 2 abgebildeten Aminosäuresequenzen. Es ist selbstverständlich möglich, die o.g. Peptide auch gentechnisch z.B. als geeignete Fusionsproteine in prooder eukaryontischen Zellsystemen herzustellen.

Reverse Transkriptase Assay. Der Mikrotest zum Nachweis der viralen Reversen Transkriptase wurde nach Gregersen et al. durchgeführt (Gregersen et al. (1988) Detection of human immunodeficiency virus and other retroviruses in cell culture supernatants by a reverse transcriptase microassay. J. Virol. Methods 19, 161-168). Zellkulturüberstände wurden durch PEG-Fällung fünffach ankonzentriert. Als positive Kontrolle (VK) dienten Überstände unbehandelter infizierter Zellkulturen, als negative Kontrolle (NK) dienten Zellkulturüberstände nicht infizierter Zellen. Der bei der NK gemessene Wert wurde verdoppelt und diente als Ausschlußwert für negative Zellen. Zur besseren Übersichtlichkeit sind die gemessenen RT-Werte in der logarithmischen Darstellung wiedergegeben (siehe auch Fig. 1, 2, 3).

### Beispiel 2: Nachweis der Inhibition der HIV-Synthese

Der Versuchsaufbau ist schematisch in der Tab. 3 wiedergegeben. Es wurden jeweils zwei Versuche zur Inhibition der HIV-Synthese mit allen zur Verfügung stehenden 41 Peptiden durchgeführt.

Bei dem ersten Versuch wurden jeweils 50 µl 1x10⁶ Jurkat-Zellen/ml in die Vertiefung von 96 Loch-Platten pipettiert und mit jeweils 50 µl (1000 TCID₅₀ HTLV-IIIB) infiziert. Die gag-Peptid-Konzentration wurde auf 200 µg/ml bzw. 40 µg/ml eingestellt. Nach einer Woche wurden 100 µl Überstand abgenommen und eventuell noch vorhandene Zellen durch Zentrifugation abgetrennt. Anschließend wurden 80 µl Überstand auf eine nicht infizierte Jurkat-Zellkultur gegeben. Der Nachweis von Virusantigen in den infizierten Zellen erfolgte mittels Immunmarkierung nach Western Blot. Hierfür wurden die nach dem Inhibitionsversuch verbleibenden Zellen in 2-fach SDS-PAGE Probenpuffer aufgenommen und in einem 14%igem PAG aufgetrennt. Danach wurden die aufgetrennten Proteine auf eine Nitrocellulosemembran geblottet und zum Nachweis viraler Proteine mit einem anti-p24 HIV-1 monoklonalen Antikörper inkubiert. Die spezifische Anfärbung erfolgte über einen zweiten anti-Maus Antikörper mit gekoppelter alkalischer Phosphatase. Die Analyse von infektiösem HIV im Überstand der Peptid behandelten Zellen erfolgte mittels Reverse Transkriptase Test.

Der zweite Versuch wurde analog dem ersten Versuchsan- satz mit einer geringeren Konzentration an infektiösen Einheiten (100 TCID₅₀) durchgeführt. Die Analyse auf infektiöses Virus im Zellkulturüberstand bzw. HIV-Protein in den infizierten Zellkulturen wurden analog dem ersten Versuch durchgeführt. In einem dritten Versuchsansatz wurden ausgewählte Peptide (2, 4, 5, 9, 28, 29), die einen inhibierenden Einfluß auf die HIV-1 Synthese ausübten, unter gleichen Bedingungen auf. ihre inhibierende Wirkung bei HIV-2 getestet. Für diesen Versuch wurden H9-Zellen mit 100 TCID₅₀ HIV-2 infiziert. Die Analyse der Infektion erfolgte analog der zwei vorhergehenden Experimente.

In den ersten beiden Experimenten wurden zwei Bereiche innerhalb der gag-Sequenz identifiziert, die jeweils durch zwei überlappende Peptide repräsentiert sind, welche einen inhibierenden Einfluß auf die HIV-Synthese ausüben. Die Peptide 4 und 5 liegen innerhalb der p17 Proteinsequenz, während 28, 29 innerhalb der p24 Proteinsequenz liegen.

In Tab. 1 sind bei dem Vergleich verschiedener HIV-gag-Proteinsequenzen die durch die Peptide 4, 5 und 28, 29 dargestellten Bereiche durch Kästchen markiert. Tab. 2 listet die Aminosäuresequenz der 41 einander überlappenden Sequenzen auf.

### Legenden:

**Tab. 1:** Vergleich verschiedener HIV-gag-Proteinsequenzen. Die durch die Peptide 4, 5 und 28, 29 dargestellten Bereiche sind durch Kästen markiert.

**Tab. 2:** Aminosäuresequenz der 41 einander überlappenden synthetischen gag-Peptide. Die Peptide wurden analog der von Ratner et al. publizierten Sequenz synthetisiert.

**Tab. 3:** Schematischer Versuchsaufbau der durchgeführten und geplanten Versuche zum Nachweis der Inhibition der HIV-Synthese durch gag-Peptide.

**Tab. 4:** Inhibition der HIV-Synthese mit gag-Peptiden. Auswertung des p24-Nachweises in HIV-infizierten Zellen nach Peptidbehandlung zwei Wochen nach Infektion. Zellen der Ausgangsplatte wurden mit 2fachen Probenpuffer versetzt und im 14%-igen PAG aufgetrennt. Nach dem Blotten auf Nitrocellulose erfolgte der Nachweis von HIV-Protein durch spezifische anti-p24 Mabs. - = keine Reaktion nachweisbar, (+) = schwache Reaktion nachweisbar, + = gute positive Reaktion nachweisbar, ++ = starke Reaktion nachweisbar.

**Fig. 1:** Inhibition der HIV Virussynthese durch gag Peptide (Versuch 1). Es wurden 1000 TCID₅₀ HIV-1 für die Infektion der Jurkat-Zellen eingesetzt. Reverse Transkriptase Aktivität gemessen in cpm im Zellkulturüberstand der Nachweiszellkultur. Der Überstand wurde durch PEG-Fällung fünffach ankonzentriert. VK = Viruskontrolle; NK = Negativ-Kontrolle. Der doppelte NK-Wert diente als Ausschluß für negative Zellkulturen. Der besseren Übersichtlichkeit wegen sind die RT-Werte in der Abbildung in der logarithmischen Darstellung wiedergegeben.

**Fig. 2:** Inhibition der HIV-Synthese durch gag Peptide (Versuch 2). Es wurden 100 TCID₅₀ HIV-1 für die Infektion der Jurkat-Zellen eingesetzt. Reverse Transkriptase Aktivität gemessen in cpm im Zellkulturüberstand der Nachweiszellkultur. Der Überstand wurde durch PEG-Fällung fünffach ankonzentriert. VK = Viruskontrolle; NK = Negativ Kontrolle. Der doppelte NK-Wert diente als Ausschluß für negative Zellkulturen. Der besseren Übersichtlichkeit wegen sind die RT-Werte in der Abbildung in der logarithmischen Darstellung wiedergegeben.

**Fig. 3:** Inhibition der HIV-Synthese durch gag Peptide (Versuch 3). Es wurden 100 TCID₅₀ HIV-1 bzw. HIV-2 für die Infektion der Jurkat bzw. H9-Zellen eingesetzt. Reverse Transkriptase Aktivität gemessen in cpm im Zellkulturüberstand der Nachweiszellkultur. Der Überstand wurde durch PEG-Fällung fünffach ankonzentriert. VK = Viruskontrolle; NK = Negativ Kontrolle. Der doppelte NK-Wert diente als Ausschluß für negative Zellkulturen. Der besseren Übersichtlichkeit wegen sind die RT-Werte in der Abbildung in der logarithmischen Darstellung wiedergegeben.

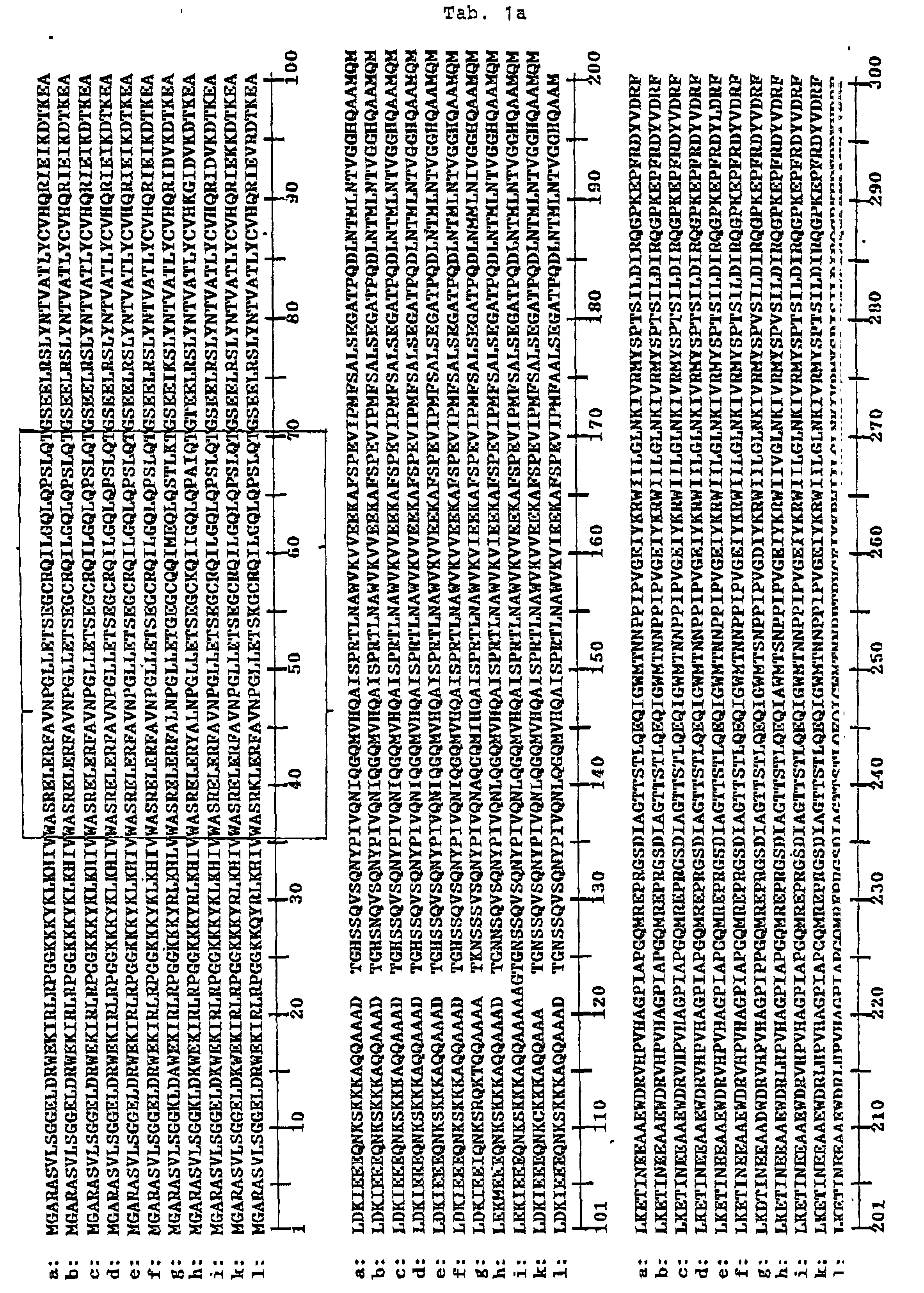

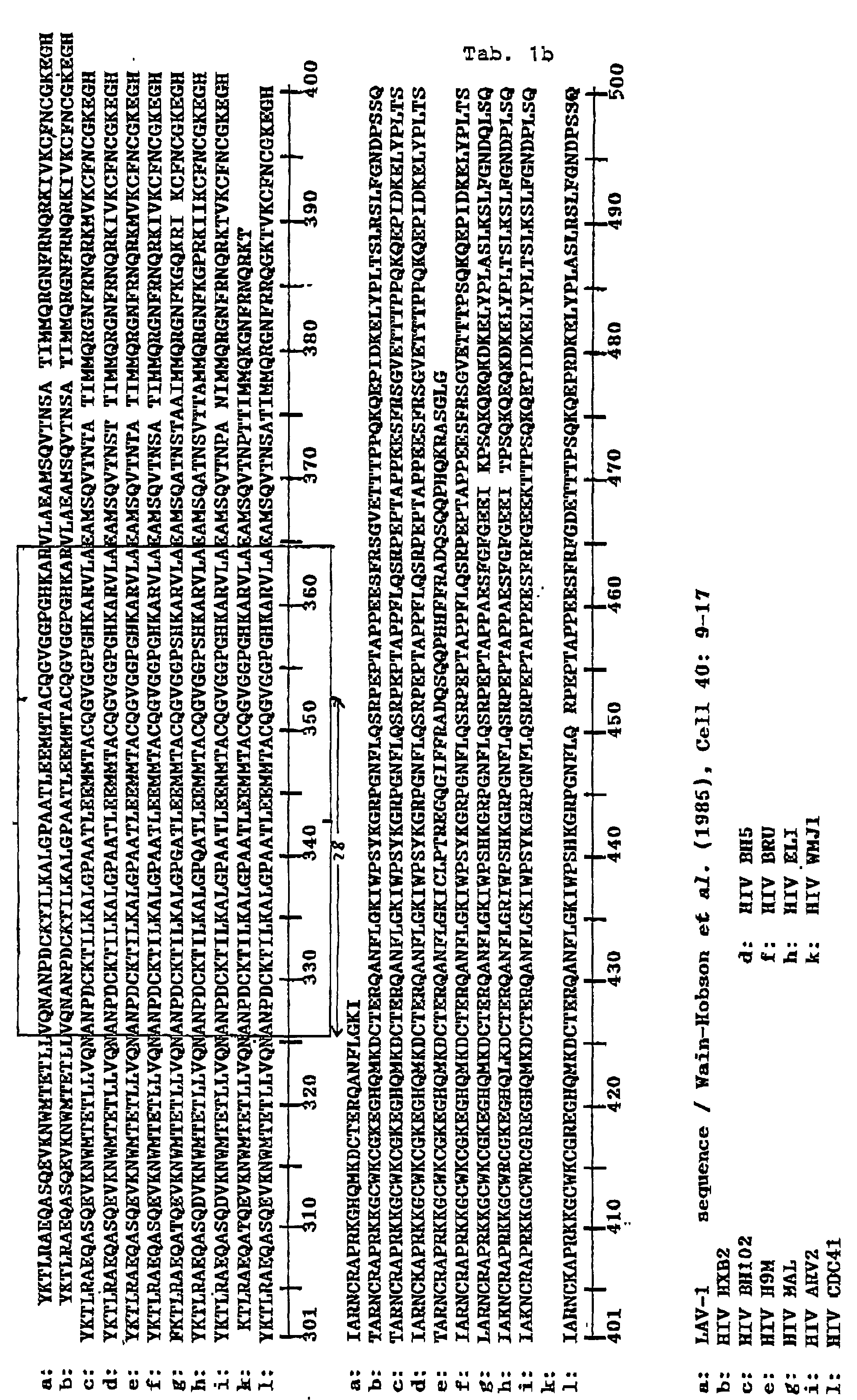

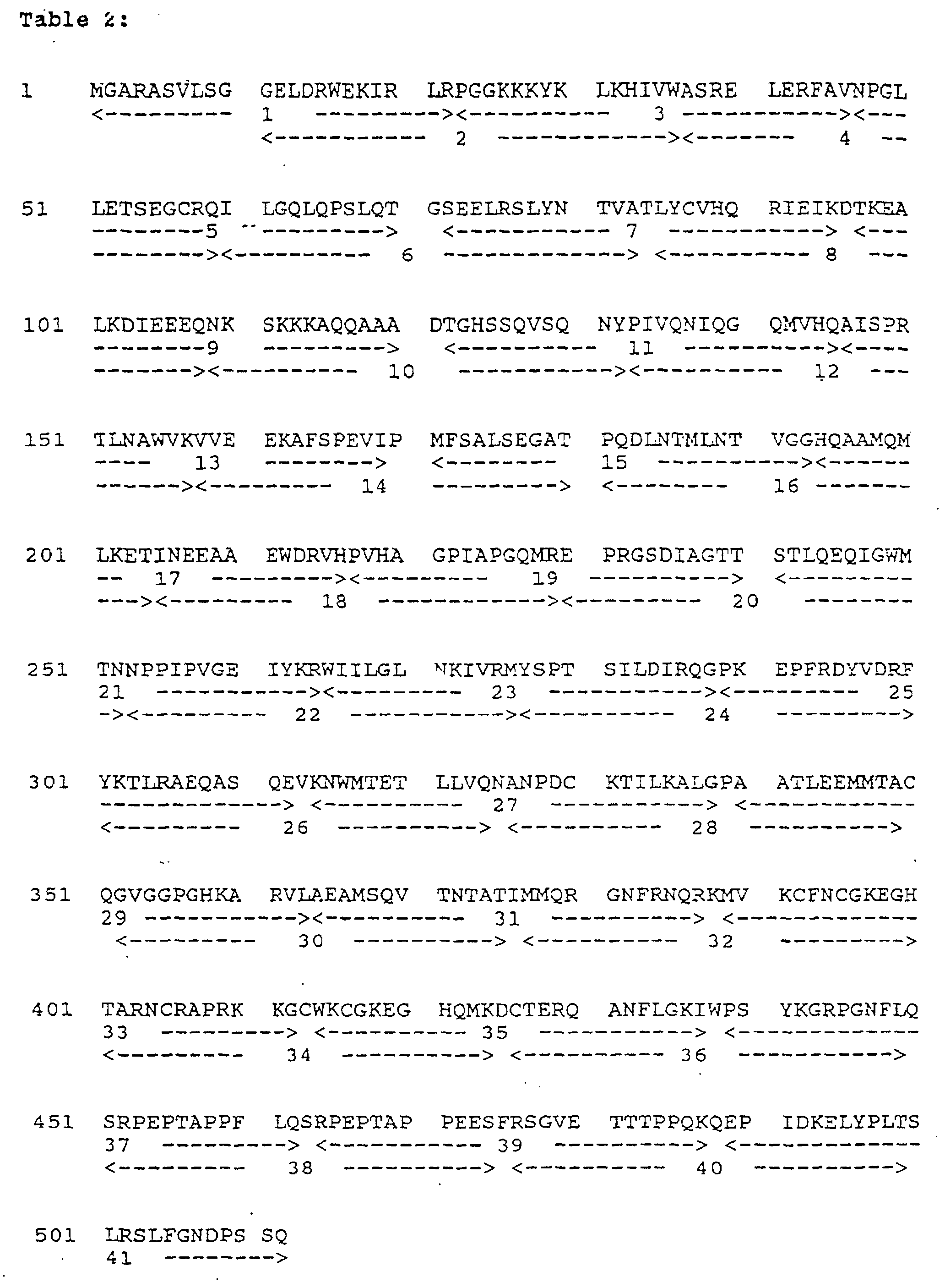

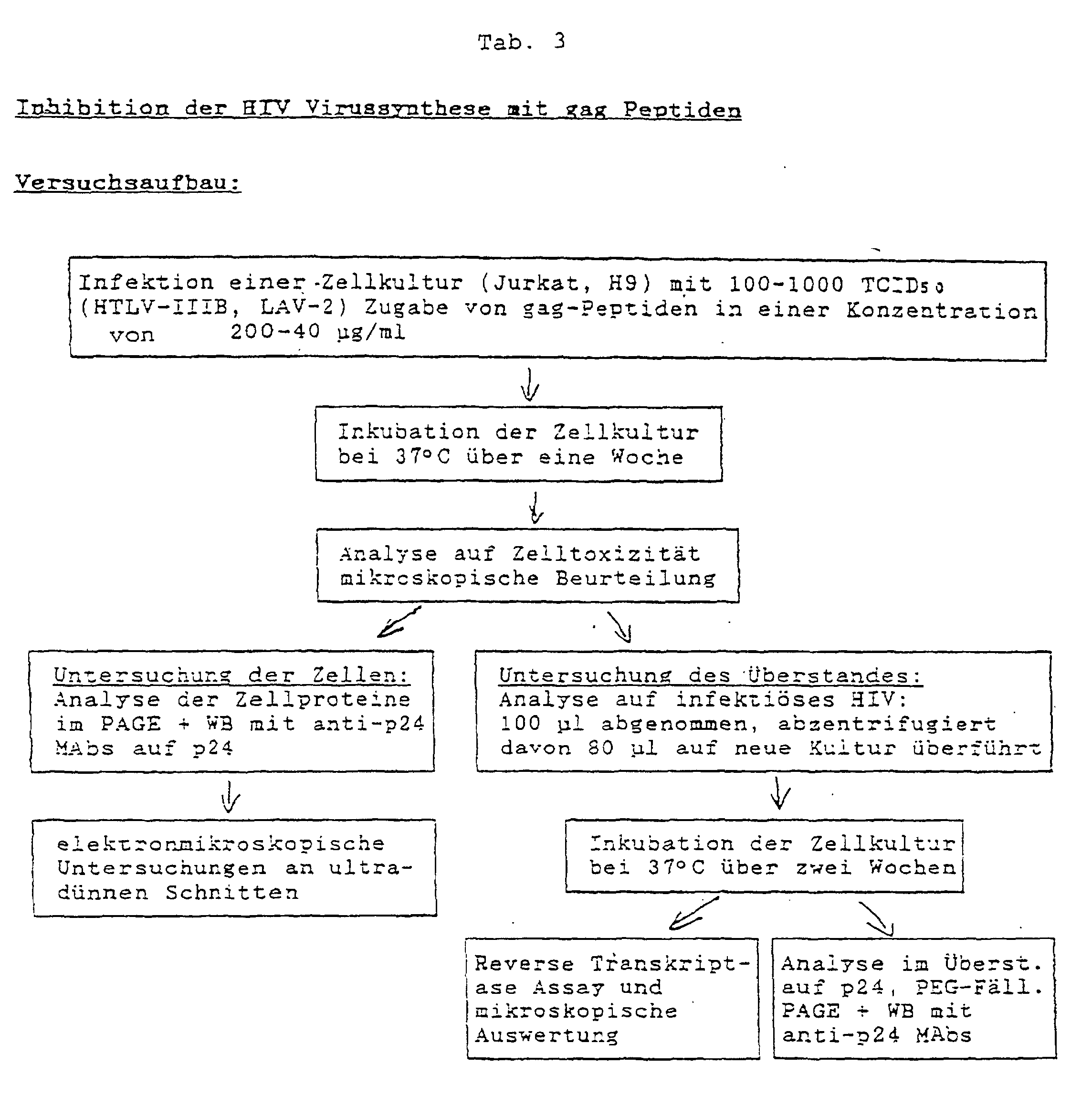

**Tab. 4**

| Inhibtion der HIV-Virussynthese mit gag-Peptiden: | |
|---|---|
| Peptid Nr.: | p24 Nachweis: |
| 1 | + |
| 2 | + |
| 3 | + |
| 4 | - |
| 5 | (+) |
| 6 | ++ |
| 7 | ++ |
| 8 | ++ |
| 9 | + |
| 10 | + |
| 11 | + |
| 12 | + |
| 13 | + |
| 14 | + |
| 15 | + |
| 16 | + |
| 17 | + |
| 18 | ++ |
| 19 | + |
| 20 | + |
| 21 | + |
| 22 | + |
| 23 | + |
| 24 | + |
| 25 | ++ |
| 26 | ++ |
| 27 | ++ |
| 28 | (+) |
| 29 | - |
| 30 | ++ |
| 31 | + |
| 32 | + |
| 33 | + |
| 34 | ++ |
| 35 | ++ |
| 36 | ++ |
| 37 | + |
| 38 | + |
| 39 | + |
| 40 | + |
| 41 | + |

## Patentansprüche (Patentansprüche für folgende(n) Vertragsstaat(en): AT, BC, CH, LI, DL, DK, FR, GB, IT, LU, NL, SE)

1. Verwendung von Peptiden des HIV-gag-Proteins, die die Peptidsequenz AATLEEMMTA enthalten und nicht größer als 50 Aminosäuren sind, zur Herstellung eines Arzneimittels zur Bekämpfung von AIDS.

2. Verwendung von Peptiden nach Anspruch 1, die die Peptidsequenz ANPDCKTILKALGPAATLEEMMTA or AATLEEMMTACQGVGGPGHKA enthalten.

3. Verwendung von Peptiden nach Anspruch 1 or 2, **dadurch gekennzeichnet, daß** die Peptidsequenz AATLEEMMTA am N- und/oder am C-Terminus um bis zu 4 Aminosäuren verkürzt ist, jedoch jeweils nicht kürzer als 6 Aminosäuren ist.

4. Verwendung von Peptiden nach mindestens einem der Ansprüche 1 bis 3, die eine oder mehrere der folgenden Aminosäureaustausche besitzen: Asparagin gegen Glutamin bzw. Glutamin gegen Asparagin, Prolin gegen Hydroxyprolin, Leucin gegen Isoleucin oder Norleucin, Glutaminsäure gegen Asparaginsäure, Threonin gegen Serin bzw. Serin gegen Threonin, Cystein gegen Serin, Arginin gegen Lysin, Alanin gegen Glycin und/oder Methionin gegen Norleucin.

5. Peptide nach mindestens einem der Ansprüche 1 bis 4 als Arzneimittel.

6. Therapeutische Zubereitung, enthaltend mindestens ein Peptid nach mindestens einem der Ansprüche 1 bis 5 zusammen mit inertem Trägermaterial.

7. Verfahren zur Herstellung der Peptide nach mindestens einem der Ansprüche 1 bis 4, wobei es sich um ein proteinchemisches Verfahren handelt, vorausgesetzt daß die Peptide nicht KALGPAATLEEMMTACQ sind.

## Patentansprüche (Patentansprüche für folgende(n) Vertragsstaat(en): GR, ES)

1. Verfahren zur Herstellung von Peptiden des HIV-gag Proteins, **dadurch gekennzeichnet, daß** die Peptide die Peptidsequenz AATLEEMMTA enthalten und nicht größer als 50 Aminosäuren sind, vorausgesetzt daß die Peptide nicht KALGPAATLEEMMTACQ sind.

2. Verfahren nach Anspruch 1, **dadurch gekennzeichnet, daß** das Peptid mit der Aminosäuresequenz ANPDCKTILKALGPAATLEEMMTA oder das Peptid mit der Aminosäuresequenz AATLEEMMTACQGVGGPGHKA enthalten ist.

3. Verfahren nach Anspruch 1 oder 2, **dadurch gekennzeichnet, daß** die Peptidsequenz AATLEEMMTA am N- und/oder am C-Terminus um bis zu 4 Aminosäuren verkürzt ist, jedoch jeweils nicht kürzer als 6 Aminosäuren ist.

4. Verfahren nach mindestens einem der Ansprüche 1 bis 3, **dadurch gekennzeichnet, daß** die Peptidsequenz AATLEEMMTA eine oder mehrere der folgenden Aminosäureaustausche besitzt: Asparagin gegen Glutamin, bzw. Glutamin gegen Asparagin, Prolin gegen Hydroxyprolin, Leucin gegen Isoleucin oder Norleucin, Glutaminsäure gegen Asparaginsäure, Threonin gegen Serin bzw. Serin gegen Threonin, Cystein gegen Serin, Arginin gegen Lysin, Alanin gegen Glycin und/oder Methionin gegen Norleucin.

5. Verfahren zur Herstellung eines Arzneimittels, **dadurch gekennzeichnet, daß** Peptide, hergestellt nach mindestens einem der Ansprüche 1 bis 4, verwendet werden.

6. Verfahren zur Herstellung eines Arzneimittels nach Anspruch 5, **dadurch gekennzeichnet, daß** zusätzlich ein inertes Trägermaterial verwendet wird.

7. Verfahren zur Herstellung eines Arzneimittels nach Anspruch 5 oder 6, **dadurch gekennzeichnet, daß** das Arzneimittel zur Bekämpfung von AIDS verwendet wird.

## Claims (Claims for the following Contracting State(s): AT, BC, CH, LI, DL, DK, FR, GB, IT, LU, NL, SE)

1. Use of peptides of the HIV gag protein which contain the peptide sequence AATLEEMMTA and are not larger than 50 amino acids for the preparation of a pharmaceutical for controlling AIDS.

2. Use of peptides according to Claim 1, which contain the peptide sequence ANPDCKTILKALGPAATLEEMMTA or AATLEEMMTACQGVGGPGHKA.

3. Use of peptides according to Claim 1 or 2, **characterized in that** the peptide sequence AATLEEMMTA is truncated by up to 4 amino acids at the N and/or at the C terminus but is not shorter than 6 amino acids in each case.

4. Use of peptides according to at least one of Claims 1 to 3, which has one or more of the following amino-acid exchanges: asparagine by glutamine, or glutamine by asparagine, proline by hydroxyproline, leucine by isoleucine or norleucine, glutamic acid by aspartic acid, threonine by serine or serine by threonine, cysteine by serine, arginine by lysine, alanine by glycine and/or methionine by norleucine.

5. Peptides according to at least one of Claims 1 to 4 as pharmaceutical.

6. Therapeutic composition containing at least one peptide according to at least one of Claims 1 to 5, together with an inert vehicle.

7. Process for the preparation of the peptides according to at least one of Claims 1 to 4, which is a process of protein chemistry, provided that the peptides are not KALGPAATLEEMMTACQ.

## Claims (Claims for the following Contracting State(s): GR, ES)

1. Process for the preparation of peptides of the HIV gag protein, **characterized in that** the peptides contain the peptide sequence AATLEEMMTA and are not larger than 50 amino acids, provided that the peptides are not KALGPAATLEEMMTACQ.

2. Process according to Claim 1, charcterized in that the peptide having the amino acid sequence ANPDCKTILKALGPAATLEEMMTA or the peptide having the amino acid sequence AATLEEMMTACQGVGGPGHKA is contained.

3. Process according to Claim 1 or 2, **characterized in that** the peptide sequence AATLEEMMTA is truncated by up to 4 amino acids at the N and/or at the C terminus but is not shorter than 6 amino acids in each case.

4. Process according to at least one of Claims 1 to 3, **characterized in that** the peptide sequence AATLEEMMTA has one or more of the following amino-acid exchanges: asparagine by glutamine, or glutamine by asparagine, proline by hydroxyproline, leucine by isoleucine or norleucine, glutamic acid by aspartic acid, threonine by serine or serine by threonine, cysteine by serine, arginine by lysine, alanine by glycine and/or methionine by norleucine.

5. Process for the preparation of a pharmaceutical, **characterized in that** peptides prepared according to at least one of Claims 1 to 4 are used.

6. Process for the preparation of a pharmaceutical according to Claim 5, **characterized in that** in addition an inert vehicle is used.

7. Process for the preparation of a pharmaceutical according to Claim 5 or 6, **characterized in that** the pharmaceutical is used for controlling AIDS.

## Revendications (Revendications pour l'(les) Etat(s) contractant(s) suivant(s): AT, BC, CH, LI, DL, DK, FR, GB, IT, LU, NL, SE)

1. Utilisation de peptides de la protéine HIV-gag, qui contiennent la séquence peptidique AATLEEMMTA, et qui n'ont pas une taille supérieure à 50 acides aminés, pour préparer un médicament destiné à lutter contre le SIDA.

2. Utilisation de peptides selon la revendication 1, qui contiennent la séquence peptidique ANPDCKTILKALGPAATLEEMMTA ou AATLEEMMTACQGVGGPGHKA.

3. Utilisation de peptides selon la revendication 1 ou 2, **caractérisée en ce que** la séquence peptidique AATLEEMMTA est, au niveau du site N- et/ou C-terminal, raccourcie d'une quantité d'acides aminés allant jusqu'à 4, mais qui cependant n'est pas raccourcie de plus de 6 acides aminés.

4. Utilisation de peptides selon au moins l'une des revendications 1 à 3, qui possèdent un ou plusieurs des remplacements d'acides aminés suivants : remplacement de la glutamine par l'asparagine ou de l'asparagine par la glutamine, de l'hydroxyproline par la proline, de l'isoleucine ou de la norleucine par la leucine, de l'acide asparagique par l'acide glutamique, de la sérine par la thréonine ou de la thréonine par la sérine, de la sérine par la cystéine, de la lysine par l'arginine, de la glycine par l'alanine et/ou de la norleucine par la méthionine.

5. Peptides selon au moins l'une des revendications 1 à 4, en tant que médicaments.

6. Préparation thérapeutique contenant au moins un peptide selon au moins l'une des revendications 1 à 5, en même temps qu'un matériau support inerte.

7. Procédé de préparation des peptides selon au moins l'une des revendications 1 à 4, **caractérisé en ce qu'**il s'agit d'un procédé de la chimie des protéines, à la condition préalable que les peptides ne soient pas KALGPAATLEEMMTACQ.

## Revendications (Revendications pour l'(les) Etat(s) contractant(s) suivant(s): GR, ES)

1. Procédé de préparation de peptides de la protéine HIV-gag, **caractérisé en ce que** les peptides contiennent la séquence AATLEEMMTA et n'ont pas une taille supérieure à 50 acides aminés, à la condition préalable que les peptides ne soient pas KALGPAATLEEMMTACQ.

2. Procédé selon la revendication 1, **caractérisé par** la présence du peptide ayant la séquence d'acides aminés ANPDCKTILKALGPAATLEEMMTA ou du peptide ayant la séquence d'acides aminés AATLEEMMTACQGVGGPGHKA.

3. Procédé selon la revendication 1 ou 2, **caractérisé en ce que** la séquence peptidique AATLEEMMTA est, au niveau du site N- et/ou C-terminal, raccourcie d'une quantité d'acides aminés allant jusqu'à 4, mais qui cependant n'est pas raccourcie de plus de 6 acides aminés.

4. Procédé selon au moins l'une des revendications 1 à 3, **caractérisé en ce que** la séquence peptidique AATLEEMMTA contient un ou plusieurs des remplacements d'acides aminés suivants : remplacement de la glutamine par l'asparagine ou de l'asparagine par la glutamine, de l'hydroxyproline par la proline, de l'isoleucine ou de la norleucine par la leucine, de l'acide asparagique par l'acide glutamique, de la sérine par la thréonine ou de la thréonine par la sérine, de la sérine par la cystéine, de la lysine par l'arginine, de la glycine par l'alanine et/ou de la norleucine par la méthionine.

5. Procédé de préparation d'un médicament, **caractérisé en ce qu'**on utilise des peptides préparés selon au moins l'une des revendications 1 à 4.

6. Procédé de préparation d'un médicament selon la revendication 5, **caractérisé en ce qu'**on utilise en outre un matériau support inerte.

7. Procédé de préparation d'un médicament selon la revendication 5 ou 6, **caractérisé en ce que** le médicament est utilisé pour lutter contre le SIDA.
